# EUROPÄISCHE PATENTSCHRIFT

(11) **EP 0 634 187 B1**
(45) Veröffentlichungstag und Bekanntmachung des Hinweises auf die Patenterteilung: **12.08.1998**
(21) Anmeldenummer: 94106469.3
(22) Anmeldetag: 26.04.1994
(51) Int. Cl.: A61M 25/00, A61B 17/22

(54) **Vorrichtung zum Auflösen von Thromben in Gefässen mittels Lyse**
Device for dissolving thrombi in vessels by lyse
Dispositif pour dissoudre des thrombis dans les vaisseaux par lyse

(30) Priorität: 17.06.1993 DE 4320150; 10.12.1993 DE 4342205
(43) Veröffentlichungstag der Anmeldung: 18.01.1995
(73) Patentinhaber: ANGIOMED GmbH & Co. Medizintechnik KG, 76227 Karlsruhe (DE)
(72) Erfinder: Starck, Erhard, Prof. Dr. med., D-61476 Kronberg/Taunus (DE); Lindenberg, Josef, D-76227 Karlsruhe (DE); Schnepp-Pesch, Wolfram, D-76185 Karlsruhe (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- EP-A- 0 378 178
- EP-A- 0 406 901
- GB-A- 2 182 567
- US-A- 5 059 178

## Beschreibung

Die Erfindung betrifft eine Vorrichtung zum Auflösen von Thromben in Gefäßen mittels Lyse nach dem Oberbegriff des Anspruchs 1.

Das Entfernen und Auflösen von Thromben geschieht derzeit derart, daß ein Katheter mit seinem vorderen, stirnseitigen, mit einer Öffnung versehenen Ende bis vor einen Thrombus geführt wird und anschließend Lyse, also Streptokinase, vom bezüglich des Arztes proximalen Ende durch den Katheter hindurch und aus dessen bezüglich des Arztes distaler vorderer Öffnung herausgedrückt wird. Es sind hierbei erhebliche Mengen von Lyse erforderlich. Die Auflösungszeit des Thrombus ist lang und beträgt 8 bis 10 Stunden.

Es ist wünschenswert, sowohl die Menge der verwendeten Lyse zu reduzieren als auch die Auflösungszeit des Thrombus zu reduzieren.

Die EP 378 178 A2 zeigt eine gattungsgemäße Vorrichtung zum Auflösen von Thromben mittels eines Lysemittels. Sie weist einen fünflumigen Katheter auf mit einem zentralen Lumen, das lediglich zur Aufnahme und Hindurchführung eines Führungsdrahtes 13 dient, der das zentrale Lumen 12 nahezu vollständig ausfüllt. Das zentrale Lumen 12 ist von einer sehr starken Wandung 14 umgeben, deren Stärke nahezu dem Durchmesser des zentralen Lumens entspricht. In dieser starken Wandung des zentralen Lumens sind um den Umfang gleichmäßig verteilt vier dünne Kanäle ausgebildet, deren Durchmesser lediglich ein Bruchteil desjenigen des zentralen Lumens und etwa ein Drittel der Wandungsstärke beträgt. Von den Kanälen erstrecken sich zu der Außenwandseite der Wandung 14 Öffnungen, die sich konisch erweitern und deren Durchmesser im Übergangsbereich zu den Kanälen etwa dem Durchmesser des Kanals entspricht, während ihr Durchmesser im Austrittsbereich, also im Umfang der Außenwandseite, größer ist.

Diese bekannte Vorrichtung kann dazu dienen, Lösungsmittel für Thromben in den Bereich der Thromben zu führen, so daß das Lösungsmittel dort auf biochemischem Wege die Thromben auflöst. Der Katheter kann in keiner Weise dazu dienen, ein mechanisches Aufbrechen und zerstören der Thromben zu ermöglichen, wodurch das biochemische Auflösen verstärkt würde.

Der Erfindung liegt daher die Aufgabe zugrunde, eine Vorrichtung zum Auflösen von Thromben zu schaffen, die auch ein mechanisches Angreifen der Thromben und damit eine schnellere biochemische Auflösung ermöglicht.

Erfindungsgemäß wird die genannte Aufgabe bei einer Vorrichtung der eingangs genannten Art durch die kennzeichnenden Merkmale des Anspruchs 1 gelöst.

Bei der erfindungsgemäßen Vorrichtung kann der Katheter bei relativ großen, das Lysemittel führenden Lumen mit einem kleinen Außenquerschnitt ausgebildet werden, so daß bei reduziertem Katheterdurchmesser ein Lösungsmittel führendes Lumen mit größerem Querschnitt erzielbar ist. Damit kann der Katheter der erfindungsgemäßen Vorrichtung in sehr feine Gefäße eingeführt werden. Die Möglichkeit, den erfindungsgemäßen Katheter sehr dünn bei relativ großem Volumen auszubilden, bedeutet eine wesentlich geringere Belastung des Patienten beim perkutanen Einführen des Katheters. Durch den relativ großen Querschnitt des Lumens im Verhältnis zum Außendurchmesser des Katheters tritt nur ein geringer Druckverlust entlang der Länge des Katheters auf, so daß das Lösungsmittel beim erfindungsgemäßen Katheter an den sehr kleinen Aussprühöffnungen mit hohem Druck ansteht und daher mit hohem Druck und hoher Geschwindigkeit aus diesen im wesentlichen radial gegen die Thromben gesprüht wird und auf diese auch mit hohem Druck auftrifft, damit tief in diese eindringt und sie mechanisch aufbricht. Hierdurch werden fluidmechanisch Spalten und Risse im Thrombus erzeugt, in die die Lyse eindringt, so daß sie an vielen Stellen und damit an einer größeren Oberfläche direkter und lokaler am Thrombenmaterial angreifen und dieses daher bei Verwendung von weniger Menge an Lyse schneller auflösen kann.

Eine erfindungsgemäße Vorrichtung wird zum Sprühen von Lyse im wesentlichen radial gegen den Thrombus und insbesondere zum mechanischen Aufbrechen desselben durch die unter hohem Druck mit hoher Geschwindigkeit im wesentlichen radial aus dem Katheter gegen den Thrombus ausgespritzte Lyse verwendet, wodurch fluidmechanisch Spalten oder Risse im Thrombus erzeugt werden, in die die Lyse eindringt, so daß sie an vielen Stellen direkter und lokaler am Thrombenmaterial angreifen und dieses daher bei Verwendung von weniger Menge an Lyse schneller auflösen kann.

Es hat sich gezeigt, daß die Lysemenge auf ein Drittel reduziert werden kann und die Auflösungszeit sich ebenfalls auf ein Drittel, d.h. ca. drei Stunden, reduziert.

Wesentlicher Kern der Erfindung ist also ein fluidmechanisches Angreifen und Aufbrechen des Thrombus und damit Erzeugen von Rissen und Spalten in diesem, über die die Lyse lokaler und direkter angreifen kann.

Gemäß einer bevorzugten Ausgestaltung ist vorgesehen, daß die Durchbrüche sich nach außen hin konisch erweitern. Hierdurch ist die Lyse in einem sich breit öffnenden Konus gegen den Thrombus spritzbar. Durch diese Ausgestaltung wird erreicht, daß zunächst durch den aus einer Öffnung austretenden Flüssigkeitskonus ein relativ großer Thrombusbereich mit der Flüssigkeit bestrahlt wird, sowohl in angularer als auch in axialer Richtung. Hierdurch wird weiterhin ermöglicht, daß eine umfassende Abdeckung der Thrombusfläche durch wenige Öffnungen oder Durchbrüche erzielbar ist. Dies bedingt wiederum, daß innerhalb des Katheters sich ein hoher Flüssigkeitsdruck aufbaut, insbesondere auch stark unterstützt dadurch, wenn die Durchbrüche bei Druckfreiheit selbstschließende Perforationen sind. Wenn die Flüssigkeit durch die Durchbrüche aus dem Katheter austritt, so sind im Flüssigkeitskonus eine Vielzahl von nadelförmigen Einzelstrahlen gebildet, da aufgrund der Konusausbildung der zunächst im Bereich des engsten Durchmessers (auf der Innenseite der Katheterwandung) gegebene Gesamtstrahl in viele nadelförmige Einzelstrahlen aufreißt, die, wie gesagt, eine sehr hohe Geschwindigkeit haben, mit der sie auf den Thrombus auftreffen, wodurch sie diesen gerade in fluid-mechanischer Weise aufbrechen können. Der Öffnungswinkel der Durchbrüche wird in der Regel im Bereich zwischen 60 und 120° gewählt, wobei eine äußerst bevorzugte Ausgestaltung sich dadurch auszeichnet, daß der Öffnungswinkel der Durchbrüche bei 90° liegt.

Weitere bevorzugte Ausgestaltungen sehen vor, daß die Durchbrüche entlang einer Schraubenlinie über den Umfang des Katheters verteilt sind. Der angulare Versatz beträgt dabei in der Regel zwischen 80 und 130°, wobei ein bevorzugter Wert 90° ist. Die Steigung der Spirale liegt üblicherweise im Bereich zwischen 0,5 und 2, wobei dieser Wert das Verhältnis von jeweils in Achsrichtung gewonnener Wegstrecke zum umlaufenden Umfang bildet, also beispielsweise den Abstand in achsparalleler Richtung miteinander fluchtender Durchbrüche zum Gesamtumfang des Katheters beinhaltet. Ein bevorzugter Wert für die Steigung ist 1. Weitere bevorzugte Ausgestaltungen sehen vor, daß der radiale Abstand benachbarter Durchbrüche zwischen 3 und 6 mm liegt und daß der axiale Abstand in achsparalleler Richtung fluchtender Durchbrüche zwischen 0,5 und 1,5 cm liegt.

Die Durchbrüche können in einfacher Weise durch Einstechen von Nadeln in die Wandung des Katheters erzeugt sein, wobei die Nadelstärke nicht mehr als 0,1 mm betragen sollt Hierdurch wird unter Berücksichtigung der Elastizität des Kathetermaterials erreicht, daß die Durchmesser der so hergestellten Durchbrüche auf jeden Fall unter den erforderlichen 0,05 mm liegen. In bevorzugter Ausgestaltung ist vorgesehen, daß die Durchbrüche bei Druckfreiheit selbstschließende Perforationen sind. Hierdurch wird erreicht, daß sich beim Eindrücken der Lyse mittels der Einrichtung zum Einspritzen, wie einer Spritze aus Zylinder und Kolben, im Inneren des Katheters ein hoher Druck aufbaut, durch den die Perforationen gegebenenfalls geöffnet werden, auf jeden Fall die Lyse unter sehr hohem Druck und mit hoher Geschwindigkeit im wesentlichen radial aus der Katheterwandung herausgespritzt wird, so daß sie den Thrombus fluidmechanisch angreifen kann.

In weiterer bevorzugter Ausgestaltung ist vorgesehen, daß der Katheter in seinem rückwärtigen bereich einen Zweiganschluß für die Einrichtung zum Einspritzen der Lyse aufweist, so daß ein gegebenenfalls zunächst verwendeter Führungsdraht zum Einführen des Katheters nicht vor Aufsetzen der Spritze bzw. der Einrichtung zum Einspritzen der Lyse entfernt werden muß.

In weiterer bevorzugter Ausgestaltung ist nämlich vorgesehen, daß ein Führungsdraht mit einem gegenüber dem Querschnitt des Lumens des Katheters geringeren Querschnitt ausgebildet ist, wobei insbesondere der Katheter an seinem rückwärtigen Ende eine Dichtung aufweist, durch die der Führungsdraht dichtend hindurchführbar ist. Durch diese, insbesondere die letztgenannte Ausgestaltung wird erreicht, daß ein an seinem vorderen Ende mit einer Öffnung versehener Katheter verwendet und dieser daher in Seldinger-Technik über einen vorher gelegten Führungsdraht bis in den Bereich des Thrombus eingeführt werden kann, wobei die Einführöffnung des Katheters durch das vordere Ende des Führungsdrahtes, welches vorzugsweise verdickt ist, zuverlässig verschlossen wird, so daß heim Einspritzen von Lyse durch den Katheter und aus den Mantelöffnungen keine Lyse aus der stirnseitigen Öffnung des Katheters austritt.

In weiterer bevorzugter Ausgestaltung kann vorgesehen sein, daß die vordere Öffnung des Katheters einen gegenüber dem Lumen des Katheters eingezogenen geringeren Querschnitt aufweist. Hierdurch wird sichergestellt, daß auch dann, wenn der Führungsdraht über seine gesamte Länge gleichen Querschnitt aufweist, zwischen der Wandung des Katheters und dem Führungsdraht ein Freiraum bleibt, durch den die Lyse geführt werden kann, und durch das vordere Ende des Führungsdrahtes die vordere Öffnung des Katheters zuverlässig verschlossen wird. In der Regel wird der Durchmesser der vorderen Öffnung des Katheters im unbelasteten Zustand geringer sein als der Querschnitt des Führungsdrahtes, so daß bei Einführen desselben in die Öffnung eine zuverlässige Dichtung erfolgt.

Eine weitere bevorzugte Ausgestaltung der Erfindung zeichnet sich dadurch aus, daß der Führungsdraht über den größten Teil seiner Länge hin einen Durchmesser von höchstens 0,05 mm aufweist, wobei weiterhin der axiale Erstreckungsbereich der Durchbrüche auf dem Mantel des Katheters beidseitig mit Begrenzungsmarkierungen versehen ist und insbesondere die Markierungen durch auf dem Mantel des Katheters angebrachte Ringe metallischer Beschichtung, vorzugsweise Platinbeschichtungen, gebildet sind.

Eine konkrete Verwendung der erfindungsgemäßen Vorrichtung sieht vor, daß ein Führungsdraht bis in den Bereich eines Thrombus eingeführt wird, anschließend der Katheter über den Führungsdraht geführt wird, bis ein vorderes Ende des Führungsdrahtes eine vordere stirnseitige Öffnung des Katheters verschließt und anschließend Lyse durch die Radialöffnungen gegen den Thrombus gespritzt wird.

Die erfindungsgemäße Vorrichtung kann ohne weiteres bei einem wandständigen Thrombus eingesetzt werden. Soweit sie bei einem obturierenden Thrombus eingesetzt werden soll, so wird man zunächst in an sich bekannter Weise einen Kanal im Thrombus erzeugen, wie durch Aufbohren oder dergleichen, in den dann der Führungsdraht und insbesondere das vordere Ende des Katheters der erfindungsgemäßen Vorrichtung eingeführt werden kann, um die erfindungsgemäße Vorrichtung zur Anwendung zu bringen.

Weitere Vorteile und Merkmale der Erfindung ergeben sich aus den Ansprüchen und aus der nachfolgenden Beschreibung, in der eine bevorzugte Ausführungsform der Erfindung unter Bezugnahme auf die Zeichnung im einzelnen erläutert ist. Dabei zeigt:
- Figur 1: ein bevorzugtes Ausführungsbeispiel der erfindungsgemäßen Vorrichtung in Seitenansicht; und
- Figur 2: eine vergrößerte Darstellung des vorderen, distalen Bereichs der erfindungsgemäßen Vorrichtung in einem Blutgefäß.

Die erfindungsgemäße Vorrichtung weist einen Katheter 1 mit einem rückwärtigen Zweiganschluß 2 auf. Am Zweiganschluß 2 ist eine Einrichtung 3 zum Einspritzen von Lyse in den Bereich eines Thrombus 4 in Form einer Spritze mit Zylinder 6 und Kolben 7 angeordnet. Der rückwärtige Hauptzweig 8 des Katheters 1 ist mit einer Dichtung (bei 9) verschlossen, durch die ein Führungsdraht 11 dicht hindurchführbar ist. Der Führungsdraht 11 kann an einer bestimmten Stelle in seinem rückwärtigen Bereich mit einer Markierung 12 versehen sein, die, wenn sie unmittelbar vor dem rückwärtigen Ende 13 des Katheters 1 angeordnet ist, anzeigt, daß die vordere Spitze 14 des Führungsdrahtes gerade eine vordere stirnseitige Öffnung 16 des Katheters 1 verschließt.

Der Katheter 1 ist in seiner Seitenwandung im vorderen Bereich mit Öffnungen oder Durchbrüchen 18 versehen, die beispielsweise schraubenförmig um den Mantel des Katheters angeordnet sein können. Die Durchbrüche oder Öffnungen 18 sind vorzugsweise als Perforationen ausgebildet, d.h. soweit im Inneren des Katheters kein höherer Druck ausgeübt wird, sind die Durchbrüche 18 unter der Eigenelastizität des Kathetermaterials geschlossen und öffnen sich nur unter Druckerhöhung im Inneren des Katheters. Die Perforationen können durch Einstechen mittels Nadeln hergestellt werden, wobei vorzugsweise Nadeln mit einem Durchmesser von nicht mehr als 0,1 mm verwendet werden, so daß unter Berücksichtigung der Eigenelastizität des Kathetermaterials die Perforationen auch im unter Druck geöffneten Zustand keinen größeren Durchmesser als etwa 0,05 mm haben.

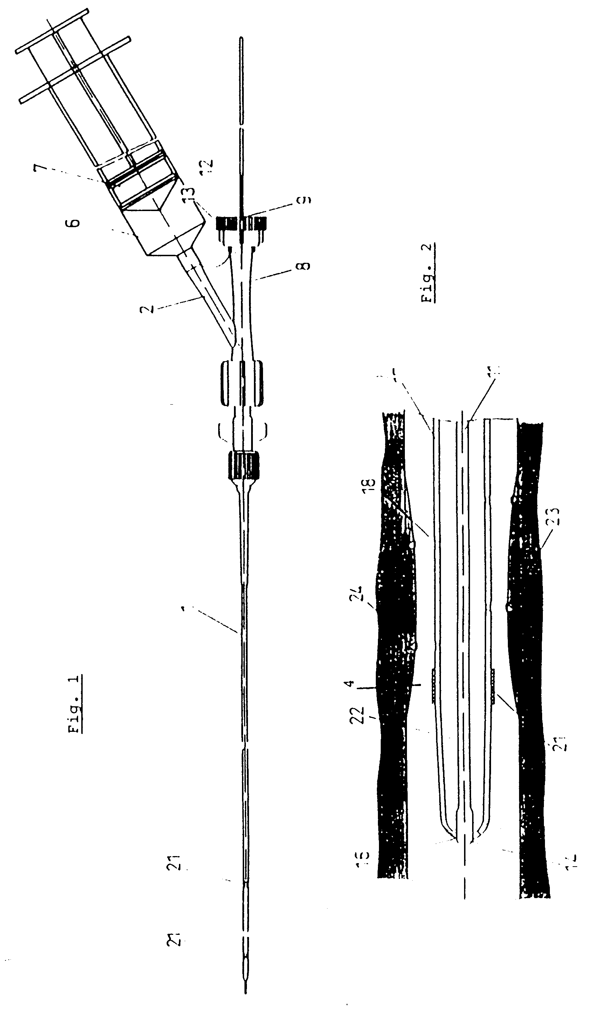

Wie insbesondere aus der Figur 2 ersichtlich ist, erweitern sich die Durchbrüche oder Perforationen 18 von der Innenseite der Katheterwandung zu deren Außenseite hin konisch. Die Lyseflüssigkeit tritt unter hohem Druck aus dem Katheter in einem breiten Öffnungskonus aus. Der Öffnungswinkel des Konus der Durchbrüche 18 beträgt im dargestellten Ausführungsbeispiel etwa 90°, kann aber auch oberhalb oder unterhalb dieses Wertes liegen. Wie aus der Figur 2 ebenfalls ersichtlich ist, liegen die Durchbrüche 18 auf dem Umfang des Katheters auf einer Schraubenlinie, deren Steigung hier etwas unter 1 beträgt. Der angulare Versatz der Durchbrüche 18 ist 90°, da durch den Führungsdraht 11 entsprechende Durchbrüche verdeckt sind.

Der Bereich der Perforationen ist zum vorderen Ende 16 und zum rückwärtigen Ende 13 hin durch Markierungen, beispielsweise dünne Platinringe 21, begrenzt. Hierdurch kann röntgenologisch die richtige Lage des vorderen Bereichs des Katheters innerhalb des Thrombus 4 festgestellt werden.

Zumindestens die Spitze bzw. das vordere Ende 14 des Führungsdrahtes 11 und die vordere stirnseitige Öffnung 16 des Katheters 1 sind derart aufeinander abgestimmt, daß das Ende 14 des Führungsdrahtes 11 die Öffnung 16 des Katheters 1 sicher verschließt, auch wenn in diesem höherer Druck ausgebildet ist. Im unbelasteten Zustand ist die vordere Öffnung 16 des Katheters 1 geringfügig kleiner als die Querabmessungen des vorderen Endes 14 des Führungsdrahtes 11, so daß dieser gut dichtend in die Öffnung 16 einführbar ist.

Der Führungsdraht 11 kann über seine gesamte Länge die gleiche Stärke aufweisen, er kann aber auch wie im dargestellten Ausführungsbeispiel einen gegenüber seinen sonstigen Querschnittsabmessungen erweiterten vorderen Bereich 14 haben. Auf jeden Fall ist es sinnvoll, daß der Katheter an seinem vorderen Ende eingezogen ist, so daß der Durchmesser der vorderen Stirnseitenöffnung 16 deutlich geringer ist als der Durchmesser des Lumens 22 des Katheters 1; so wird sichergestellt, daß in jedem Falle einerseits die vordere Öffnung 16 durch den Führungsdraht 11, 14 verschließbar ist, andererseits im Inneren des Katheters 1 zwischen der Wandung 17 und dem Führungsdraht 11 ein freier Bereich bleibt, durch den die Lyse hindurchgeführt werden kann.

Der Einsatz der erfindungsgemäßen Vorrichtung in der dargestellten bevorzugten Ausführungsform geschieht folgendermaßen:

Zunächst wird der Führungsdraht 11 in üblicher Weise in der Regel perkutan bis in den Bereich des Thrombus 4 eingeführt. Anschließend wird in ebenfalls üblicher Weise der Katheter 1 in Seldinger-Technik über den Führungsdraht 11 unter Röntgen- oder unter Ultraschallbeobachtung soweit eingeführt, bis er mit seinem Bereich, in dem die Perforationen 18 angeordnet sind, im Inneren des Thrombus 4 zu liegen kommt, wobei dies über die Markierungen 21 kontrolliert wird. Aufgrund vorheriger Beobachtung über die Länge des Thrombus 4 kann ein geeigneter Katheter ausgewählt werden, bei dem die Länge, über die die Perforationen 18 angeordnet sind, d.h. der Abstand zwischen den Markierungen 21, der Länge des Thrombus entspricht.

Im weiteren wird dafür gesorgt, daß die vordere Öffnung 16 des Katheters 1 geschlossen wird, indem diese soweit über das vordere Ende des Führungsdrahtes 11 geschoben bzw. letzterer mit seinem vorderen Ende in die Öffnung 16 eingeschoben oder eingezogen wird, daß die gegebenenfalls erweiterte Spitze 14 des Führungsdrahtes 11 sich in der Öffnung 16 befindet und diese verschließt.

Falls nicht schon vorher geschehen, kann nun am Zweig 2 des Katheters 1 eine mit Lyse gefüllte Spritze angesetzt werden. - Alternativ kann vor Überführen des Katheters 1 über den Führungsdraht 11 bei angesetzter Spritze auch schon durch den Katheter Lyse in die Spritze 3 eingesogen worden sein.

Auf jeden Fall wird die Lyse in der Spritze 3 mittels des Kolbens 7 nun durch den Katheter 1 und aus den Öffnungen 18 herausgedrückt, wobei die Lyse aus den Öffnungen 18 aufgrund des ausgeübten hohen Drucks unter hoher Geschwindigkeit in feinen Strahlen austritt. Hierdurch wird der Thrombus 4 an den Auftreffstellen 23 aufgebrochen, so daß Risse 24 entstehen, in die die Lyse eintritt. Sie kann damit wesentlich direkter einwirken, als dies mit den bisher bekannten Vorrichtungen und dem bisher bekannten Vorgehen der Fall ist. Durch die Erfindung kann der Einsatz von Lyse bei einem gegebenen Thrombus auf ein Drittel reduziert werden. Die Auflösezeit wird ebenfalls wesentlich reduziert und zwar auch auf ein Drittel, beispielsweise von 8 bis 10 Stunden auf lediglich 3 Stunden.

## Patentansprüche

1. Vorrichtung zum Auflösen von Thromben in Gefäßen mittels eines Lysemittels, mit einem insbesondere perkutan einführbaren Katheter (1), mit einer Einrichtung (2, 6, 7) zum Einspritzen des Lysemittels durch den Katheter in den Bereich des Thrombus, wobei im vorderen Bereich des Katheters (1) in dessen Wandung Durchbrüche (18) vorgesehen sind, durch die das Lysemittel im wesentlichen radial gegen den Thrombus (4) spritzbar ist, dadurch gekennzeichnet, daß der Katheter (1) aus elastischem Material besteht, daß der Katheter einlumig ist und seine Wandung (17) nur eine Stärke mit einem Bruchteil des Querschnitts des Lumens (22) aufweist, daß das distale Ende des Katheters (1) verschließbar oder geschlossen ist und daß die Durchbrüche (18) einen Herstellungs-Durchmesser unterhalb 0,05 mm aufweisen.

2. Vorrichtung nach Anspruch 1, dadurch gekennzeichnet, daß der Katheter (1) ein elastischer Katheter ist und daß die Durchbrüche (18) als bei Druckfreiheit selbstschließende Perforationen ausgebildet sind.

3. Vorrichtung nach Anspruch 1 oder 2, dadurch gekennzeichnet, daß der Katheter (1) in seinem rückwärtigen Bereich einen Zweiganschluß (2) für die Einrichtung zum Einspritzen der Lyse aufweist.

4. Vorrichtung nach einem der vorangehenden Ansprüche, gekennzeichnet durch einen Führungsdraht (11) mit einem gegenüber dem Querschnitt des Lumens (22) des Katheters (1) geringeren Querschnitt.

5. Vorrichtung nach Anspruch 4, dadurch gekennzeichnet, daß der Katheter (1) an seinem rückwärtigen Ende (13) eine Dichtung (9) aufweist, durch die der Führungsdraht (11) dichtend hindurchführbar ist.

6. Vorrichtung nach einem der vorangehenden Ansprüche, dadurch gekennzeichnet, daß das vordere Ende des Katheters (1) eine Öffnung (16) aufweist, die durch das vordere Ende (14) eines Führungsdrahts (11) verschließbar ist.

7. Vorrichtung nach Anspruch 6, dadurch gekennzeichnet, daß die vordere Öffnung (16) des Katheters (1) einen gegenüber dem Lumen (22) des Katheters (1) eingezogenen geringeren Querschnitt aufweist.

8. Vorrichtung nach einem der Ansprüche 4 bis 7, dadurch gekennzeichnet, daß der Führungsdraht an seinem vorderen Ende eine Erweiterung (15) gegenüber seinem Querschnitt im rückwärtigen Bereich aufweist.

9. Vorrichtung nach einem Ansprüche 4 bis 8, dadurch gekennzeichnet, daß der Führungsdraht über den größten Teil seiner Länge hin einen Durchmesser von höchstens 0,05 mm aufweist.

10. Vorrichtung nach einem der vorangehenden Ansprüche, dadurch gekennzeichnet, daß der axiale Erstreckungsbereich der Durchbrüche (18) auf dem Mantel (17) des Katheters (1) beidseitig mit Begrenzungsmarkierungen (21) versehen ist.

11. Vorrichtung nach Anspruch 10, dadurch gekennzeichnet, daß die Markierungen durch auf dem Mantel (17) des Katheters (1) angebrachte Ringe metallischer Beschichtung, vorzugsweise Platinbeschichtungen, gebildet sind.

12. Vorrichtung nach einem der vorangehenden Ansprüche, dadurch gekennzeichnet, daß die Durchbrüche (18) sich nach außen hin konisch erweitern.

13. Vorrichtung nach Anspruch 12, dadurch gekennzeichnet, daß der Öffnungswinkel der Durchbrüche (18) zwischen 60 und 120° beträgt.

14. Vorrichtung nach Anspruch 12 oder 13, dadurch gekennzeichnet, daß der Öffnungswinkel der Durchbrüche (18) bei 90° liegt.

15. Vorrichtung nach einem der vorangehenden Ansprüche, dadurch gekennzeichnet, daß die Durchbrüche (18) entlang einer Schraubenlinie über den Umfang des Katheters (1) verteilt sind.

16. Vorrichtung nach einem der vorangehenden Ansprüche, dadurch gekennzeichnet, daß der angulare Versatz benachbarter Durchbrüche zwischen 80 und 130° liegt.

17. Vorrichtung nach Anspruch 15, dadurch gekennzeichnet, daß die Steigung der Schraubenlinie zwischen 0,5 und 2 liegt.

18. Vorrichtung nach einem der vorangehenden Ansprüche, dadurch gekennzeichnet, daß der angulare Abstand benachbarter Durchbrüche (18) zwischen 3 und 6 mm liegt.

19. Vorrichtung nach einem der vorangehenden Ansprüche, dadurch gekennzeichnet, daß der axiale Abstand in achsparalleler Richtung fluchtender Durchbrüche (18) zwischen 0,5 und 1,5 cm liegt.

## Claims

1. Apparatus for dissolving thrombi in vessels by means of a lysing agent, having an in particular percutaneously introduceable catheter (1), having a device (2, 6, 7) for injecting the lysing agent through the catheter into the area of the thrombus, where in the front area of the catheter (1) and namely in the wall thereof openings (18) are provided through which the lysing agent can be injected substantially radially against the thrombus (4), characterized in that the catheter (1) is made from elastic material, that the catheter is in single-channel form and its wall (17) only has a thickness representing a fraction of the cross-section of the channel (22), that the distal end of the catheter (1) is sealable or sealed and that the openings (18) have a manufacturing diameter below 0.05 mm.

2. Apparatus according to claim 1, characterized in that the catheter (1) is an elastic catheter and that the openings (18) are constructed as perforations which self-seal in the case of freedom from pressure.

3. Apparatus according to claim 1 or 2, characterized in that the rear area of the catheter (1) has a branch connection (2) for the lysing agent injection device.

4. Apparatus according to one of the preceding claims, characterized by a guide wire (11) with a smaller cross-section than that of the channel (22) of the catheter (1).

5. Apparatus according to claim 4, characterized in that at its rear end (13), the catheter (1) has a seal (9) through which the guide wire (11) can be passed in sealing manner.

6. Apparatus according to one of the preceding claims, characterized in that the front end of the catheter (1) has an opening (16), which is sealable by the front end (14) of a guide wire (11).

7. Apparatus according to claim 6, characterized in that the front opening (16) of the catheter (1) has a drawn-in smaller cross-section compared with the channel (22) of the catheter (1).

8. Apparatus according to one of the claims 4 to 7, characterized in that at its front end the guide wire has a widening (15) compared with its cross-section in the rear area.

9. Apparatus according to one of the claims 4 to 8, characterized in that, over most of its length, the guide wire has a diameter of max 0.05 mm.

10. Apparatus according to one of the preceding claims, characterized in that the axial extension area of the openings (18) on the jacket (17) of the catheter (1) is provided on both sides with boundary markings (21).

11. Apparatus according to claim 10, characterized in that the markings are formed by metallic coating, preferably platinum coating rings applied to the jacket (17) of the catheter (1).

12. Apparatus according to one of the preceding claims, characterized in that the openings (18) widen conically outwards.

13. Apparatus according to claim 12, characterized in that the opening angle of the openings (18) is between 60 and 120°.

14. Apparatus according to claim 12 or 13, characterized in that the opening angle of the openings (18) is 90°.

15. Apparatus according to one of the preceding claims, characterized in that the openings (18) are distributed along a helix over the circumference of the cathether (1).

16. Apparatus according to one of the preceding claims, characterized in that the angular displacement of adjacent openings is between 80 and 130°.

17. Apparatus according to claim 15, characterized in that the pitch of the helix is between 0.5 and 2.

18. Apparatus according to one of the preceding claims, characterized in that the angular spacing of adjacent openings (18) is between 3 and 6 mm.

19. Apparatus according to one of the preceding claims, characterized in that the axial spacing in axial parallel direction of aligned openings (18) is between 0.5 and 1.5 cm.

## Revendications

1. Dispositif pour dissoudre des thrombi dans des vaisseaux, au moyen d'un produit lysogène, comportant un cathéter (1), en particulier susceptible d'être introduit de façon percutanée, comportant un dispositif (2, 6, 7) permettant d'injecter le produit lysogène, à travers le cathéter, dans la zone du thrombus, des perçages (18) étant prévus, dans la zone avant du cathéter (1), dans les parois de celui-ci, à travers lesquels le produit lysogène est susceptible d'être injecté sensiblement radialement contre le thrombus (4), caractérisé en ce que le cathéter (1) est constitué en matériau élastique, en ce que le cathéter présente une lumière et sa paroi (17) présente une épaisseur de seulement une fraction de la section transversale de la lumière (22), en ce que l'extrémité distale du cathéter (1) est susceptible d'être obturée ou est fermée, et en ce que les perçages (18) présentent un diamètre de fabrication inférieur à 0,05 mm.

2. Dispositif selon la revendication 1, caractérisé en ce que le cathéter (1) est un cathéter élastique et en ce que les perçages (18) sont conformés en perforations se fermant automatiquement en cas d'absence de pression.

3. Dispositif selon la revendication 1 ou 2, caractérisé en ce que le cathéter (1), dans sa zone arrière, présente un raccord ramifié (2) destiné au dispositif d'injection pour la lyse.

4. Dispositif selon l'une des revendications précédentes, caractérisé par un fil-guide (11) présentant une section transversale inférieure à la section transversale de la lumière (22) du cathéter (1).

5. Dispositif selon la revendication 4, caractérisé en ce que le cathéter (1), à son extrémité arrière (13), présente un joint (9) à travers lequel le fil-guide (11) peut être introduit de façon étanche.

6. Dispositif selon l'une des revendications précédentes, caractérisé en ce que l'extrémité avant du cathéter (1) présente une ouverture (16) susceptible d'être obturée par l'extrémité avant (14) d'un fil-guide (11).

7. Dispositif selon la revendication 6, caractérisé en ce que l'ouverture avant (16) du cathéter (1) présente une section transversale rétractée et plus réduite que le lumen (22) du cathéter (1).

8. Dispositif selon l'une des revendications 4 à 7, caractérisé en ce que le fil-guide présente, à son extrémité avant, un élargissement (15) par rapport à la section transversale qu'il présente dans la zone arrière.

9. Dispositif selon l'une des revendications 4 à 8, caractérisé en ce que le fil-guide, sur la plus grande partie de sa longueur, présente un diamètre maximum de 0,05 mm.

10. Dispositif selon l'une des revendications précédentes, caractérisé en ce que la zone d'extension axiale des perçages (18), sur l'enveloppe (17) du cathéter (1), est pourvue, de part et d'autre, de marquages de délimitation (21).

11. Dispositif selon la revendication 10, caractérisé en ce que les marquages présents sur l'enveloppe (17) du cathéter sont formés par des bagues d'un revêtement métallique, de préférence en platine.

12. Dispositif selon l'une des revendications précédentes, caractérisé en ce que les perçages (18) vont en s'élargissant de façon conique en direction de l'extérieur.

13. Dispositif selon la revendication 12, caractérisé en ce que l'angle d'ouverture des perçages (18) est compris entre 60 et 120°.

14. Dispositif selon la revendication 12 ou 13, caractérisé en ce que l'angle d'ouverture des perçages (18) est approximativement de 90°.

15. Dispositif selon l'une des revendications précédentes, caractérisé en ce que les perçages (18) sont répartis, selon une hélice, sur la circonférence du cathéter (1).

16. Dispositif selon l'une des revendications précédentes, caractérisé en ce que le décalage angulaire de perçages adjacents est compris entre 80 et 130°.

17. Dispositif selon la revendication 15, caractérisé en ce que le pas de l'hélice est compris entre 0,5 et 2.

18. Dispositif selon l'une des revendications précédentes, caractérisé en ce que l'écart angulaire entre des perçages (18) adjacents est compris entre 3 et 6 mm.

19. Dispositif selon l'une des revendications précédentes, caractérisé en ce que l'écart axial entre des perçages (18) alignés dans la direction parallèle à l'axe est compris entre 0,5 et 1,5 cm.
